(19) Europäisches Patentamt

European Patent Office

Office européen des brevets

(11) Publication number: **0 490 839 A2**

## (12) EUROPEAN PATENT APPLICATION

(21) Application number: **91830547.5**

(22) Date of filing: **09.12.91**

(51) Int. Cl.5: **G01N 33/546**, //G01N33/58, G01N33/76,G01N33/78

(30) Priority: **13.12.90 IT 4856890**

(43) Date of publication of application:
**17.06.92 Bulletin 92/25**

(84) Designated Contracting States:
**AT BE CH DE DK ES FR GB GR LI LU MC NL SE**

(71) Applicant: **De Vizia, Antonio**
**34 Via Casale Sanfelice**
**I-83030 Montefusco AV(IT)**
Applicant: **Federici, Giorgio**
**24 Via Rodolfo Lanciani**
**I-00162 Roma RM(IT)**
Applicant: **Tavarone, Vito**
**4 Via Gaetano Salvemini**
**I-82100 Benevento BN(IT)**

(72) Inventor: **De Vizia, Antonio**
**34 Via Casale Sanfelice**
**I-83030 Montefusco AV(IT)**
Inventor: **Federici, Giorgio**
**24 Via Rodolfo Lanciani**
**I-00162 Roma RM(IT)**
Inventor: **Tavarone, Vito**
**4 Via Gaetano Salvemini**
**I-82100 Benevento BN(IT)**

(74) Representative: **Bazzichelli, Alfredo et al**
**c/o Società Italiana Brevetti S.p.A. Piazza di Pietra, 39**
**I-00186 Roma(IT)**

(54) **Method and kit for enzyme-immunoassay.**

(57) The present invention relates to a method for the enzymoimmunologic determination of analytes from solutions based on the antibody-antigen mechanism, in which after having fixed certain reagents (antibodies) on microbeads having a high specific surface, the antibody-antigen reaction is made to take place, and the reaction products are filtered away from the excess reagents, determination of the analyte to be determined being performed on the excess reagents only. A further object of the present invention is determination kits for performing said method.

EP 0 490 839 A2

FIG. 1

DETERMINATION OF $T_4$ IN THE FILTRATE

EP 0 490 839 A2

The present invention relates to a method for the enzymoimmunologic determination of analytes from solutions and determination kits for its performance.

The analytic and quantitative methods using antibodies and antigens as primary reagents have at the present time become part of routine practice and are indispensable both in clinical and pharmacological research and in basic research.

The application of values determined by RIA (Radioimmunoassay) revolutionized, during the 'Sixties, analytical research in various fields of research, causing a revolution to various aspects of biology, clinical chemistry and other subjects. However the need to make use of adequate radio-protection processes and the production of a notable quantity of radioactive waste have caused a limitation in the use of said procedures. Another limitation has been caused by the limited halflife of the reagents used in RIA techniques. These conditions have caused a turn towards research after alternative methods for labeling the components used in immunochemical methods, with particular reference to immunologic determination. As a consequence of this research, over the last few years there has been a proliferation of a large number of methods based on non-isotopical labeling methods. In some cases simply by transposition from isotopical marking to immunoenzymatic labeling and spectrophotometrical reading; in other cases through the development of new methods of detection often more sensitive than the RIA techniques themselves.

A number of methods have been proposed and developped, based on fluorescence detections, both in phase and polarized and also by means of the use of luminescent compounds and by means of the use of electrochemical sensors or biosensors.

The spectophotometrical techniques which form an alternative to the radioimmunological techniques, in spite of the fact that they have reduced environmental risks, lowered costs and simplified the instrumentation, show, however, several disadvantages. These disadvantages are connected with the instability of the chromophore substrates used, and above all with the possible interference in spectrophotometrical readings between signals coming from the reagents and signals coming from the reaction products, in consideration of the fact that said measurements are made in the presence of both compounds. Similar difficulties have been found in the electrochemical readings proposed as an alternative to spectrphotometrics. In this type of reading one obtains the combination of a specificity for a substrate by an enzyme, along with the high sensitivity of electrochemical methods. The electrochemical methods have been employed with success in the determination of the activity of redox enzymes. The enzymatic labeling agent catalyzes a redox reaction and the product formed can be determined on the surface of a measuring electrode. However, in order to increase the sensitivity of these assays, it has been preferred to use enzymes which can generate an electrochemically active product from an electrochemically inactive, and therefore non-interfering, substrate (or substrates). From the concentration of the electrochemically active product it is then possible to determine the concentration of the analyte to be determined. In the literature is described (Analytical Chemistry 1984, 56, 2355 to 2360) the use of alcaline phosphatase (EC 3.1.3.1) using the phenylphosphate substrate which is transformed into the electrochemically active compound phenol. Phenol can be revealed from its oxydation at + 670 mV against Ag/AgCl. However this method cannot generally speaking be used in a system containing components which oxydate at potentials close to this, and this circumstance makes it of little practical use in the determination of compounds contained in samples of blood or serum, also due to the simultanous presence in the measuring solution of reagents and reaction products which can cause mutual interference (similar to that which takes place in spectrophotometric determination), which is principally shown by a decrease in sensitivity of the method, or by a "swamping" of the signal measured at the electrode, which is due to the electrochemically active product generated by an electrochemically inactive substrate by substances present in solution. In the state of the art, a system is described in which the alkaline phosphatase as an enzyme label, catalyzes the conversion of phenylphosphate in phenol; the phenol, generated as a reaction product, before being detected up by an electrode is separated on a column of octyl-decylsilane in order to prevent the signal caused by the phenol (which is directly related to the concentration of the analyte to be determined) from being swamped or influenced by the discharge of other components of the solution. This system has not been found to be suitable, due to its complexity, for determinations which involve a large number of samples.

It has now been surprisingly found that the above described disadvantages can be overcome by the use of a method of separating reaction products and excess reagents, by means of fixing certain reagents to a support and filtering, which makes it possible to determine the analyte to be determined from the excess reagents alone.

Object of the present invention is therefore a method for the enzymoimmunologic determination of immunoenzymatic analytes from solutions, comprising the following operations:

a) binding an antibody specific to the analyte to be determined on a support, and introduction of said support antibody bound to the support into the reaction solution;

3

b) introducing the analyte to be determined into said reaction solution;

c) adding to said reaction solution a conjugate containing an enzyme and an antigen identical to said analyte or an antibody different from the antibody bound to said support, said conjugate being at a higher concentration both than the concentration of said antibody bound to the support, and than the concentration of said analyte;

d) maintaining said solution for the time required for a reaction between said antibody bound to the support and/or said analyte and/or said conjugate with the formation of antibody-antigen complexes bound to said support;

e) separating the unreacted substances from the reaction solution and their collection;

f) adding to said unreacted substances a substrate which can be transformed by said enzyme of said conjugate into an active form for a system of measurement and determination of their concentration characterized in that

i) said support is formed by microbeads having a high specific surface of a a diameter not exceeding 6 mm, and

ii) said separating operation e) is performed by filtering on porous filters having a pore diameter such as not to allow the passage of said complexes, but only the passage of the unreacted substances.

A further object of the present invention are determination kits for the performance of determinations according to the method of the present invention.

Under the expression immunoenzymatic analytes in the present invention are to be understood analytes which can be qualitatively detected and quantitatively determined through reactions based on the mechanism antibody-antigen and through the use of labeling enzymes.

The method according to the present invention is aimed at the determination of analytes in solution using antigen antibody techniques (in which at least one antibody is fixed to a support). Said method can be used for the qualitative and quantitative determination of analytes of the most varied origin.

In the following will be described two methods based on the antigen-antibody technique. In the first, called the competition method, a quantity of natural antigen (the analyte to be determined) competes with a predetermined quantity of the same antigen labeled with an enzyme (conjugate) for a limited number of antibody sites fixed to a support.

In this case the concentration of the conjugate is greater both than the concentration of the analyte and than that of the antibody sites, and the latter is less than the sum of the concentrations of the analyte and the conjugate. In the other method, called the sandwich method, to a solution containing a specific antibody for an analyte to be determined said specific antibody being bound to a support, are added the analyte to be determined and a second antibody labeled with enzyme (conjugate). The second antibody results specific for the analyte to be determined for a different epitope from that for which the first antibody is specific, and is present in a concentration greater than both the analyte to be labeled and the first antibody fixed to the support.

In both cases, once the reaction has taken place, with the formation of an antibody/antigen and antibody/antigen labeled with enzyme complex in the case of the competition method, and of an antibody fixed to a support/antigen to be determined/second antibody labeled with enzyme complex in the case of the sandwich method, filtration of the excess products is carried out. This filtration is performed on plates (using filtering under pressure if necessary) and the solution collected is additioned with a substrate which is transformed into an electrochemically or spectrophotmetrically active form, the concentration of which is then determined by means of electrochemical sensors or spectrophotometers. From the concentration of the substrate that is transformed by the enzyme it is possible to calculate the concentration of the enzyme in the filtrate. From this concentration it is possible to determine, by means of a calibration curve, the concentration of the natural antigen to be determined. The calibration curve can also be linearized, optimizing it according to the interval of phisiological concentration of the analyte to be determined.

In both methods it is necessary in any case, before determining the analyte, to determine the saturation concentration of the substrate-fixed antibody. In the field of application of the present invention, the use of alkaline phosphatase has been shown to be particularly advantageous both in electrochemical and in spectrophotometrical determinations, as has $\beta$-D-galactosidase. 1-naphtyl-phosphate is added to the alkaline phosphatase (in the case of electrochemical determinations) with the formation of 1-naphtole according to the following reaction diagram:

1-naphtyl-phosphate + alkaline phosphatase → 1-naphtole

1-naphtole being the electrochemically active product; and, in the case of spectrophotometric determinations, the substrate p-nitrophenyl-phosphate is added according to the following reaction diagram:

4

p-nitrofenilphosphate + alkaline phosphatase → p-nitrophenol

which is the spectrophotometrically active product.

As regards β-D-galactosidase, β-naphtyl-β-D-galactopyranoside is used as a substrate, with formation of 1-naphtole according to the following diagram:

β-naphtyl-β-D-galactopyranoside + β-D-galactosidase → 1-naphtole

which is the electrochemically active product.

Electrochemical determination is performed by the use of electrochemical cells containing a reference electrode and an electrode comprising carbon particles having a particle diameter of 30-50 nanometers which include a metal from the platinum group. The carbon particles can be bound using a resin or other binding agent. It has been shown to be particularly advantageous to set a potential between the electrodes in such a way as to obtain the redox potential of the 1-naphtole at a value of +300 mV. The reference electrode can be formed by any sort of conventional reference electrode, for example Ag/AgCl. It is possible to include a system of electrochemical cells to make simultaneous multiple determinations easier, following for example the printed circuit plate described in application W 086/03837. However, preferred electrodes include an electrically conductive porous layer of bound carbon or graphite particles, a metal of the platinum group finely subdivided, for example platinum or palladium, which are thoroughly mixed with them or deposited, absorbed on the particles before proceeding with the binding, for example using a resin, to form the electrically conductive porous layer. The production and characteristics of the preferred electrodes are described in the USA patents 4044193, 4166143, 4293396 and 4478696.

Spectrophotometric determination is performed using instruments known to experts in this field, for example microplate readers made by BIOTEK (USA) or the like.

The choice of materials to form the microbeads to be used in the process according to the present invention is not critical, and a large number of materials can be chosen, on condition that they be inert to all the reaction components. To increase surface activity it is preferred, according to the present invention, to use microbeads having a diameter not exceeding 6 mm.

The method and determining kit according to the present invention can be easily adapted to immunotests on substances of clinical importance and those of none, coupled with alkaline phosphatase and β-D-galactosidase.

The present invention will be described in greater detail by means of examples, which are not intended as limitations, and with reference to the enclosed drawings, in which

figure 1 is a graph (current change versus concentration) showing the determination of $T_4$ following the competition method with electrochemical detection in the filtrate;

figure 2 is a graph (optical density versus concentration) showing the determination of $T_4$ following the competition method with spectrophotometric detection in the filtrate,

figure 3 is a graph (current change versus concentration) showing the determination of TSH following the sandwich method in the filtrate in the presence of an excess of a second antibody labeled with an enzyme; and

figure 4 is a graph (current change versus concentration) showing the determination of TSH following the sandwich method with electrochemical detection (in the filtrate and, as a control, on the plate containing the microbeads) in the presence of a slight excess of a second antibody labeled with an enzyme.

Example 1

Competition method

A monoclonal (or polyclonal) anti-T4 (tyroxine or tetraiodotyronine) antibody was immobilized on particles of agarosium following a method for fixing the protein to the support based on activation using cyanogen bromide. 10 ml of Ultrogel ACA22 (agarosium) were washed in a Buckner funnel with 3,4 volumes of distilled water. The gel was dried out gently, eliminating the supernatant only, and was re-suspended in 40 ml of deionized water under moderate stirring. Keeping the solution under stirring and setting the thermostat at 18°C, the pH of the suspension was adjusted to 10,5-11 by addition of NaOH 4M. 10 ml of a solution of cyanogen bromide at a concentration 1 g/ml in acetonitrile are then added, and a moderate stirring is continued for 8-10 minutes, readjusting the pH to a value between 10,5 and 11. The activated gel is then washed rapidly with 100 ml of deionized water, then with 20 ml of acetone containing 5% water and finally with 500 ml of bicarbonate buffer solution 0,1 M at pH 9,5. The activated gel is transfered into a flask containing 20 ml of bicarbonate buffer solution 0,1 M at pH 8,3 with NaCl 0,5 M. The solution is moderately stirred and is additioned with 5 ml of the binding solution (that is to say the anti-T4 antibody) containing 0,5-1 mg per ml of protein. The stirring is continued for 2-4 hours at room temperature, or all night at 4°C. The gel is then washed using the same buffer solution used for binding of the antibody, performing this washing with high ionic strength buffer with the object of eliminating all the non-specific ionic interactions. The gel is then treated with a volume of ethanolamine 0,1 M buffered at pH 8,0 to block the access of activated groups that have not reacted. This treatment must go on for at least 3 hours at 4°C. The gel is then washed with tris-HCl buffer 0,01 M pH 8,5 containing 1 M of magnesium chloride. The gel suspension thus obtained is kept at 4°C. At the same time, the $T_4$ conjugate (tyroxine)-alkaline phosphatase is prepared using tyroxine (Sigma) and alkaline phosphatase (Boehringer). To a solution of 10 mg of tyroxine in 2 ml of methanol containing 250 g/liter of N-ethylmorfoline, 6 mg of dimethyladipimate are added, the whole is mixed in a Vortex and left at room temperature for 30 minutes. 0,6 mg of alkaline phosphatase are then added in 2 ml of sodiumcarbonate buffer 0,1 M at pH 9,9 containing $MgCl_2$ 10 M, and the mixture is left for 90 minutes at room temperature. Separation of the conjugate obtained from the eccess tyroxine molecules which have not reacted is obtained through gel filtration on a 1,2 x 26 cm column of Sephadex G25 fine grade using a tris-acetate 50 mM buffer solution at pH 7,5 as an eluent and containing $MgCl_2$ 10M.

As an alternative, the conjugate of tyroxine (T4) is also prepared with $\beta$-D-galactosidase.

To 10 mg of tyroxine (T4) in 2 ml of methanol containing 250 g/liter of N-ethylmorpholine, 6 mg of dimethyl-adipimate are added, the mixture is stirred and left for 10 minutes at room temperature. To 100 microliters of this solution are added 1 ml of sodium carbonate buffer solution pH 9,9 (containing $MgCl_2$ 10 mM) and mercaptoethanol also containing 140 microgrammes of $\beta$-D-galactosidase.

The solution is stirred and left at room temperature for 90 minutes, then the reaction is blocked by adding 1 ml of tris-acetate buffer at pH 7,5 containing 10 mM of $MgCl_2$ and 10 mM of mercaptoethanol. The separation of the conjugate is performed on a 1,2 x 25 column of fine Sephadex 0,25 using as an eluent the same buffer solution used to block the coupling reaction between tyroxine and alkaline phosphatase. Alternatively, using the conjugate between tyroxine and alkaline phosphatase or that with $\beta$-D-galactosidase the quantity of antibody which has coupled with the microbeads is measured. To 100 microliters of microbeads (Ultrogel, diameter $200\mu$) containing approximately 5000 microbeads, are added graded quantities of enzyme/analyte conjugate in a predetermined addition scale with the object of evaluating the maximum binding capacity. This reaction is performed on wells containing microfiltration apparatuses (Millipore, model Millipack) with Durapore membranes in polyvinylidendifluoride with diameters of 0,10, 0,22, 0,45, 0,65 microns. The result is followed using two methods. After an incubation lasting 30 minutes filtering is carried out using the system with vacuum pump (provided with the Millipack apparatus). In these conditions it is possible to measure the efficiency of the conjugate bond adding the substrates for alkaline phosphatase (or for $\beta$-D-galactosidase) both in the filtrate and in the upper part of the plate where the microbeads with bonded conjugate remain. In the first case a growing activity can be detected once saturation of the microbeads has been reached. In the second case, on the other hand, there is growing activity until saturation is reached, after which activity remains constant even when adding an excess of conjugate.

In the following table 1 is shown the activity of alkaline phosphatase in the filter system after immobilization of an anti-$T_4$ antibody on microbeads and competition with $T_4$ labeled with alkaline phosphatase. The incubation time is 30 minutes and after filtering the activity is determined using 1-naphtylphosphate. Saturation is performed on approximately 5000 microbeads in tris-HCl buffer solution at pH 10,2. As can be seen from the table, saturation is reached using 40 $\mu$l of conjugate.

TABLE 1

| T$_4$ alkaline phosphatase | Filtrate (corr. in $\mu$A) | Microbeads (corr. in $\mu$A) |
|---|---|---|
| 0 | 0,40 | 0,39 (white) |
| 10 | 0,39 | 1,8 |
| 20 | 0,41 | 2,4 |
| 30 | 0,40 | 3,7 |
| 40 | 0,42 | 7,5 |
| 50 | 2,4 | 7,4 |
| 60 | 4,3 | 7,6 |
| 70 | 6,9 | 7,5 |

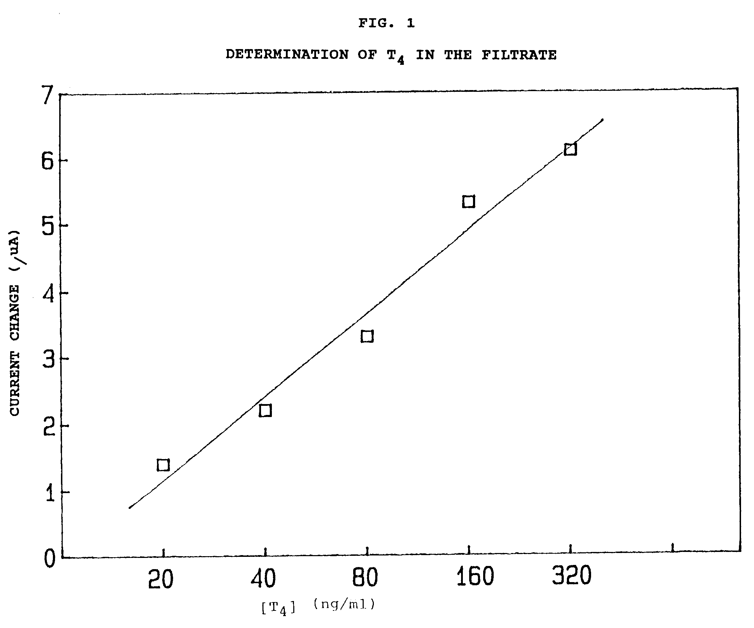

Having determined in this way the quantity of microbeads with immobilized antibody (saturation concentration) and that of analyte coupled with the enzyme to be used one passes on to the determination of analytes in biological samples using the naftilphosphate substrate. The reaction mixture deposited in each well of the Millipack plate contains 25 microliters of sample or of standard solution, 100 microliters of microbead suspension and 100 microliters of tyroxine conjugated with enzyme. After an incubation period of 30 minutes, filtering is carried out. During filtering the substrate is added (in this case 1-naphtyl-phosphate dissolved in 50 microliters of buffer and the reaction is made to continue for 15 minutes. After this, the amperometric reading is carried out. The results are summarized in figure 1.

Determination of T$_4$, as given in example 1 and in figure 1, can also be performed using the classical methods described in the literature for the photometrical determination of the alkaline phosphatase conjugated enzyme. In this case it is necessary to proceed according to example 1 until filtration. The photometric substrate must then be added; this is p-nitrophenylphosphate.

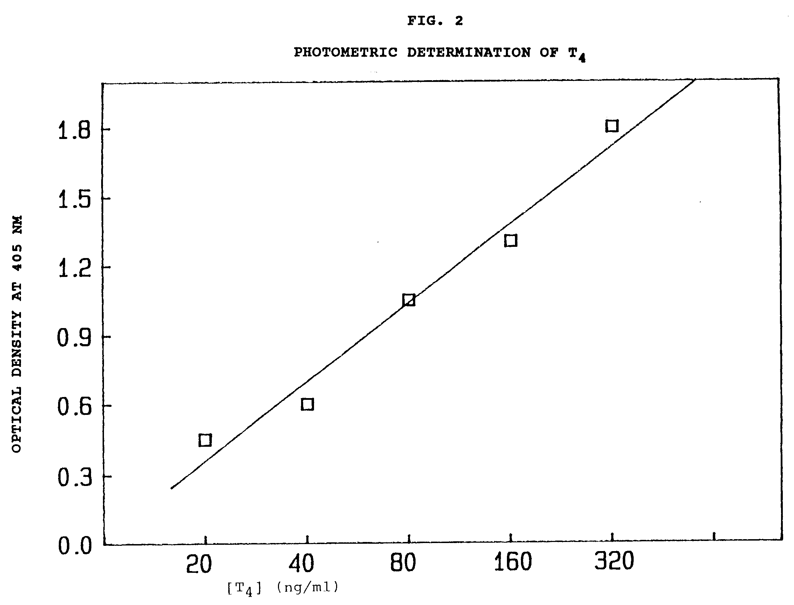

To the filtrate are added 100 $\mu$l of p-nitrophenylphosphate at a concentration of 2 mg/ml in diethanolamine-HCl 0,1 M buffer at pH 9,8 containing 50 mM magnesium chloride. The mixture is left to incubate at room temperature for 15 minutes, necessary for development of the reaction, then the reaction is blocked by the addition of 50 $\mu$l of NaOH 3 N. Absorbance is read at 405 nm and the results set down in graph form (fig. 2) as in example 1.

Example 2

Sandwich method for the determination of TSH

In this example two monoclonal antibodies are used which are specific for different epitopes of TSH. The first antibody is bound to the microbeads of Ultrogel following the same method based on cyanogen bromide as an activating agent used in example 1. The second antibody, on the contrary, is used coupled to the enzyme alkaline phophatase or $\beta$-D-galactosidase. In the case of the second antibody being labeled with alkaline phosphatase, the method using glutaraldehyde as a binding agent is followed, and it is necessary to proceed as follows. The monoclonal antibody, at a concentration of 2 mg/ml and the alkaline phosphatase (5 mg) with activity greater than 1000 U/ml are dialyzed together against a phosphate buffer at pH 7,4 0,1 M, changing the buffer at least four times. After dialysis the solution is removed from the dialysis tube and the volume is measured. The amount of glutaraldehyde needed is represented by a final concentration of 2% volume/volume. This calculated amount of glutaraldehyde is added slowly to the mixture of antibody-enzyme, keeping the reaction container under stirring at 4°C, and incubation is made to continue under continuous light stirring for 2 hours. The mixture is then transferred into a dialysis tube and dialized for 12 hours against phosphate buffer at pH 7,4, changing the buffer twice. The dialysis bag is then transferred into another recipient and dialyzed again for 2 hours at 4°C against two changes of tris 0,5 M buffer at pH 8,0. The mixture is then removed from the dialysis bag and diluted to 4 ml using the same tris buffer containing 1% bovine albumine serum.

For the labeling of the second antibody with $\beta$-galactosidase, a method using maleimmide as a binding agent is used. A solution of monoclonal antibody at a concentrations of 3 mg/ml in sodium acetate 0,1 M buffer at pH 5,0 is prepared. 2 ml of the antibody solution are added dropwise to a saturated solution of N,N'-phenylendimaleimide in sodium acetate buffer at pH 5, 0,1 M. The mixture is incubated at 30°C for 20 minutes; the solution is then fractioned on a column of Sephadex G25 pre-balanced with the same reaction buffer. The fractions containing the antibody are collected. The pH of these fractions is then adjusted to 6,5

7

EP 0 490 839 A2

using phosphate buffer pH 7,5 0,5M. Simultaneously, the $\beta$-D-galactosidase is dialyzed against a sodium phosphate buffer at pH 7,0 10 mM. The antibody treated with D-maleimide (0,5-1,0 mg) is incubated with 0,5 mg of $\beta$-D-galactosidase at 30° for 20 minutes in a final volume of q ml of sodium-acetate buffer pH 5,0. At the end of the incubation the pH is adjusted to 7,0 and the preparation is chromatographed on a column of Sepharosio 6B (1,5 x 40 cm) which has been balanced in precedence using 10 mM sodium phosphate buffer containing 0,1 M of NaCl, 1 mM of $MgCl_2$ and 0,01% of bovine serum albumine using the same buffer for elution. Fractions of 0,7 ml are collected and the absorption of each fraction at 280 nm measured. Each fraction is then diluted 50-300 times and the enzymatic activity present in 10 microliters is measured. From these values it is possible to determine the enzyme/antibody ratio present in the complex.

With a method equivalent to the one described in example 1 the quantity of saturation of the first antibody/microbead complex is measured using, for a specified quantity of microbeads (approximately 5000) an excess of TSH and the second antibody labeled with alkaline phosphatase or $\beta$-D-galactosidase. On obtaining this result the system can be used for determination of TSH present in biological samples by means of two methods. The first method requires the presence of a large excess of second antibody labeled with enzyme, whereas the second one requires the presence of a slight excess of second antibody labeled with enzyme.

Excess of second antibody marked with enzyme

100 microliters of biological or standard sample were incubated in the wells of the Millipack plate, adding 0,1 ml of microbeads suspension with the first antibody bound and 50 microliters of the second antibody coupled to the enzyme. 50 microliters contains a quantity of second antibody which is four times the first antibody. After 30 minutes of incubation, filtration is performed and 0,1 ml of substrate are added, for electrochemical determination. After 15 minutes of reaction the electrodic system is inserted directly into the Millipack plate.

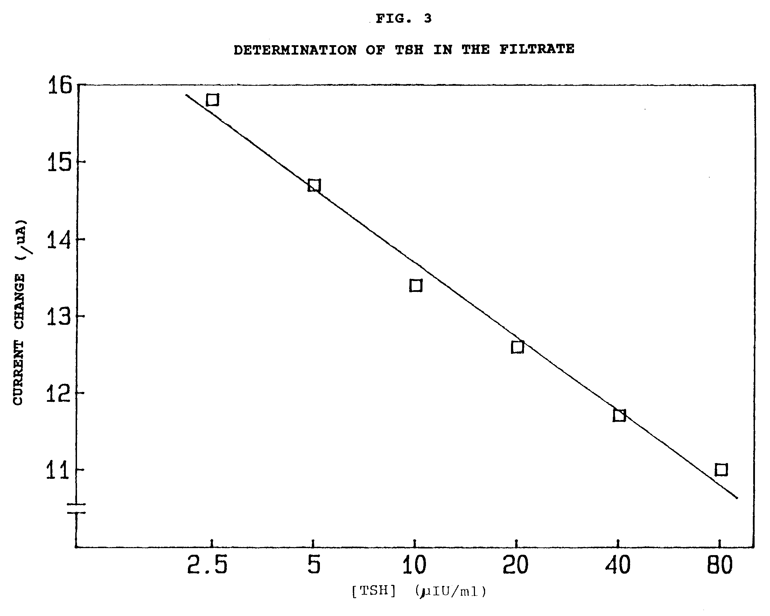

The results are shown in figure 3.

Slight excess of second antibody labeled with enzyme.

In this case incubation is performed as in the previous case, with the difference that in the 50 microliters the second antibody is only in excess by 1,3 with respect to the first antibody. After 30 minutes of incubation, filtering is carried out. In this specific case the reading can be performed both on the filtrate and on the Millipack plate. The filtrate is additioned with a suitable amount of substrate for the enzyme and after 15 minutes of incubation a reading is taken with the electrochemical sensor, or else substrate and buffers are added to the wells containing the microbeads and in this case also measurements are taken using the electrochemical sensor.

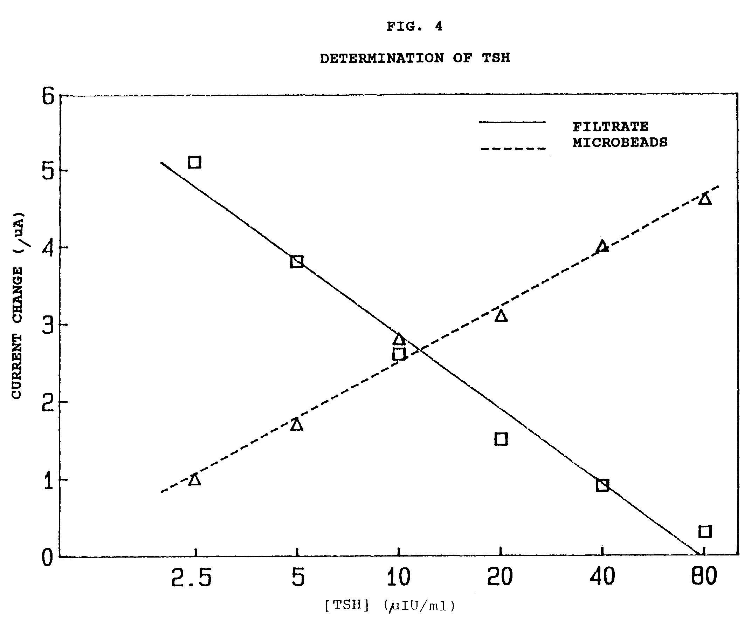

The results are given in figure 4, in which the reading on the filtrate is also given as a comparison.

An advantageous alternative to the method described in the preceding examples is represented by the possibility of fixing an antigen to the gel support, in this case developing a method for determination of an antibody. In this case an excess, with respect to the concentration of the fixed antigen, of an antibody labeled with enzyme (alkaline phosphatase or $\beta$-D-galactosidase) and an antibody of natural origin, are additioned going on to perform filtering and reading on the filtrate.

The following gives the parts forming two typical kits, the first of which uses the competition method and the second, the sandwich method.

Kit for the competition method

    1. Filtration plate with 96 wells (base made of polyvinylidendifluoride).
    2. Plate with 96 wells for collection of filtrate.
    3. Set of calibrators (from 2 to 6, the number depending on the type of analyte to be determined and its physiological concentration)
    4. Calibrator with pre-defined concentration.
    5. Control serum (liophile) at known concentration.
    6. Flask containing the antigen-enzyme conjugate.
    7. Flask containing the specific antibodies attached to the microbeads.
    8. Flask containing the substrate.

Kit for the sandwich method

In the case of the sandwich method the equipment is identical to that given hereabove, except that the flask containing the antigen-enzyme conjugate is substituted by a conjugate formed from the second antibody conjugated with an enzyme.

**Claims**

1.  A method for the enzymoimmunologic determination of immunoenzymatic analytes from solutions, comprising the following operations:

    a) binding an antibody specific to the analyte to be determined on a support, and introducing said antibody bound to the support into the reaction solution;

    b) introducing the analyte to be determined into said reaction solution;

    c) adding to said reaction solution a conjugate containing an enzyme and an antigen identical to said analyte or an antibody different from the antibody bound to said support, said conjugate being at a higher concentration both than the concentration of said antibody bound to the support, and than the concentration of said analyte;

    d) maintaining said solution for the time required for a reaction between said antibody bound to the support and/or said analyte and/or said conjugate with the formation of antibody-antigen complexes bound to said support;

    e) separating the unreacted substances from the reaction solution and their collection;

    f) adding to said unreacted substances a substrate which can be transformed by said enzyme of said conjugate into an active form for a system of measurement and determination of their concentration

    characterized in that

    i) said support is formed by microbeads having a high specific surface of a diameter not exceeding 6 mm and

    ii) said separating operation e) is performed by filtering on porous filters having a pore diameter such as not to allow the passage of said complexes, but only the passage of said unreacted substances.

2.  Method according to claim 1, in which said conjugate added in operation c) is formed by a substance identical to the analyte to be determined, bound to an enzyme, whereby in said operation d) a first complex is formed between said antibody bound to the support and said analyte and a second complex between said antibody bound to the support and said conjugate.

3.  Method according to claim 1, in which said conjugate added in operation c) is formed by a second antibody for the analyte to be determined, said second antibody being specific for an epitope of said analyte different from the epitope for which is specific the antibody bound to the support and being bound to an enzyme, whereby in said operation d) a single complex is formed, made up of said antibody bound to the support, said analyte to be determined and said second antibody bound to an enzyme.

4.  Method according to any one of the preceding claims in which said microbeads have a diameter not exceeding 1 mm.

5.  Method according to claims from 1 to 4, in which in operation f) the determination of the concentration of said analyte is performed electrochemically.

6.  Method according to claims from 1 to 4, in which in operation f) the determination of the concentration of said analyte is performed spectophotometrically.

7.  Enzymoimmunological determination kit for the determination of immunoenzymatic analytes from solutions using the competition method as claimed in claim 2, comprising

    a) filtration plate for filtration;

    b) plate with wells for collection of filtrate;

    c) set of calibrators;

    d) calibrator with pre-defined concentration;

    e) control serum (liophile) at known concentration;

    f) flask containing the analyte-enzyme conjugate;

g) flask containing the specific antibody for the analyte to be determined bound to the microbeads;

h) flask containing the substrate.

8. Enzymoimmunologic determination for the determination of immunoenzymatic analytes from solutions using the method as claimed in claim 3, comprising

a) filtration plate for filtration;

b) plate with chambers for collection of filtrate;

c) set of calibrators; d) calibrator with pre-defined concentration;

e) control serum (liophile) at known concentration;

f) flask containing the second antibody-enzyme conjugate;

g) flask containing the specific antibody for the analyte bound to the microbeads;

h) flask containing the substrate.

FIG. 1

DETERMINATION OF $T_4$ IN THE FILTRATE

FIG. 2

PHOTOMETRIC DETERMINATION OF $T_4$

OPTICAL DENSITY AT 405 NM

[$T_4$] (ng/ml)

12

FIG. 3

DETERMINATION OF TSH IN THE FILTRATE

CURRENT CHANGE (/uA)

[TSH] (μIU/ml)

FIG. 4

DETERMINATION OF TSH

EP 0 490 839 A2

14